# EUROPÄISCHE PATENTSCHRIFT

(11) **EP 3 649 919 B1**
(45) Veröffentlichungstag und Bekanntmachung des Hinweises auf die Patenterteilung: **01.01.2025**
(21) Anmeldenummer: 18205618.4
(22) Anmeldetag: 12.11.2018
(51) Int. Cl.: A61B 1/24, A61B 5/00, G06T 17/00

(54) **DENTALES BILDAUFNAHMESYSTEM**
DENTAL IMAGING SYSTEM
SYSTÈME D'IMAGERIE DENTAIRE

(43) Veröffentlichungstag der Anmeldung: 13.05.2020
(73) Patentinhaber: Ivoclar Vivadent AG, 9494 Schaan (LI)
(72) Erfinder: FOLLONIER, Stéphane, 7324 Vilters (CH); WACHTER, Wolfgang, 9494 Schaan (LI)
(74) Vertreter: Baldus, Oliver

(56) Entgegenhaltungen:
- EP-A1- 2 010 045
- WO-A1-2018/030969
- US-A1- 2013 236 850
- US-A1- 2016 220 105
- US-A1- 2018 168 781

## Beschreibung

Die vorliegende Erfindung betrifft ein dentales Bildaufnahmesystem und ein Verfahren zur dentalen Bildaufnahme.

Die Druckschrift US 2016/220105 A1 betrifft ein Gerät zum Betrachten des Inneren eines Mundes. Ein LED-, OLED-, Halogen-, Plasma- oder Laser-Lichtsystem projiziert auf den Zahn eine Strahlung in Form von Punkten, Linien oder bekannten und strukturierten Rahmen. Durch ein Verändern von Wellenlängen und Intensitäten können pathologische Bereiche untersucht werden.

Die Druckschrift US 2018/168781 A1 betrifft einen Scanner, eine Augmented-Reality-Anzeige und ein Berechnungsgerät. Durch das AR-System kann beispielsweise ein realer Zahn in einer unterschiedlichen Farbe in einer visuellen Überlagerung hervorgehoben werden.

Die Druckschrift WO 2018/030969 A1 betrifft ein Beleuchtungsgerät für eine Bildgebung im Dentalbereich, das an einem Smartphone befestigt werden kann.

Die Druckschrift US 2013/236850 A1 betrifft einen intraoralen Scanner zur digitalen Bildaufnahme mit einer Kollimatorlinsengruppe zum Kollimieren des Lichtstrahls.

Das bildliche Erfassen einer Zahnsituation im Inneren einer Mundhöhle unter Lumineszenz kann schwierig sein, da sich Lumineszenzbereiche bei Tageslicht oftmals nur schwer erkennen lassen.

Es ist die technische Aufgabe der vorliegenden Erfindung, verbesserte Bilddaten für eine zahnmedizinische Diagnose bereitzustellen.

Diese Aufgabe wird durch Gegenstände nach den unabhängigen Ansprüchen gelöst. Vorteilhafte Ausführungsformen sind Gegenstand der abhängigen Ansprüche, der Beschreibung und der Figuren.

Das Bildaufnahmesystem kann eine Lichtquelle zum Anregen einer Lumineszenz im Inneren einer Mundhöhle umfassen. Die Anregung der Lumineszenz von Zahnsubstanz kann mit oder ohne zusätzlichen Farbstoff durchgeführt werden. Durch das dentale Bildaufnahmesystem wird der technische Vorteil erreicht, dass der Lumineszenzbereich besser optisch wahrgenommen werden kann.

In einer technisch vorteilhaften Ausführungsform des Bildaufnahmesystems umfasst das dentale Bildaufnahmesystem eine Berechnungseinrichtung, die ausgebildet ist, einen Kontrast oder eine Intensität des erfassten Lumineszenzbereiches im digitalen Bild zu erhöhen. Dadurch wird beispielsweise der technische Vorteil erreicht, dass Lumineszenzbereiche noch deutlicher optisch wahrgenommen werden können. Durch die bildliche Überlagerung auf dem beleuchteten Zahn durch die Datenbrille lassen sich Lumineszenzbereich in Echtzeit darstellen und bei einer medizinischen Untersuchung unmittelbar erkennen.

In einer weiteren technisch vorteilhaften Ausführungsform des Bildaufnahmesystems umfasst das dentale Bildaufnahmesystem eine Berechnungseinrichtung, die ausgebildet ist, eine Tiefeninformation in das digitale Bild zu integrieren, um die Basis des digitalen Bildes zu erhöhen. Dadurch wird beispielsweise ebenfalls der technische Vorteil erreicht, dass Lumineszenzbereiche noch deutlicher optisch wahrgenommen werden können oder dass eine Tiefe der kariösen Läsion angezeigt werden kann.

In einer weiteren technisch vorteilhaften Ausführungsform des Bildaufnahmesystems ist eine Lichtquelle zum Anregen einer Lumineszenz und/oder die elektronische Kamera in der Datenbrille oder in einer dentalen Behandlungsvorrichtung integriert. Dadurch wird beispielsweise der technische Vorteil erreicht, dass die Lichtquelle an geeigneten Positionen gehalten werden kann.

In einer weiteren technisch vorteilhaften Ausführungsform des Bildaufnahmesystems ist die Lichtquelle ausgebildet, monochromatisches, nicht-monochromatisches, nicht-polarisiertes oder polarisiertes Licht auszusenden. Dadurch wird beispielsweise der technische Vorteil erreicht, dass geeignetes Licht zur Erfassung der Lumineszenz verwendet wird. Das Licht kann kontinuierlich ausgesendet oder gepulst ausgesendet werden.

Das monochrome Filter, das Bandpassfilter oder das Polarisationsfilter wird elektronisch gesteuert.

In einer weiteren technisch vorteilhaften Ausführungsform des Bildaufnahmesystems weist das Licht eine Wellenlänge im Bereich von 300 bis 480 nm auf. Das Licht kann jedoch auch eine Wellenlänge im Bereich von 330 bis 400 nm aufweisen. Das Licht kann beispielsweise eine Wellenlänge von 244 nm, 257 nm, 325 nm, 364 nm im Ultraviolettbereich, eine Wellenlänge von 457 nm, 473 nm, 488 nm, 514 nm, 532 nm, 633 nm, 660 nm im sichtbaren Bereich oder eine Wellenlänge von 785 nm, 830 nm, 980 nm, 1064 nm im Infrarotbereich aufweisen. Dadurch wird beispielsweise der technische Vorteil erreicht, dass sich die Lumineszenz von Zahnsubstanz besonders gut anregen lässt.

In einer weiteren technisch vorteilhaften Ausführungsform des Bildaufnahmesystems umfasst die Lichtquelle ein monochromes Filter, ein Bandpassfilter oder ein Polarisationsfilter. Dadurch wird beispielsweise der technische Vorteil erreicht, dass sich die Bildaufnahme der Kamera verbessern lässt.

In einer weiteren technisch vorteilhaften Ausführungsform des Bildaufnahmesystems ist das monochrome Filter, das Bandpassfilter oder das Polarisationsfilter bedarfsweise in den Strahlengang der Lichtquelle bewegbar.

Dadurch wird beispielsweise der technische Vorteil erreicht, dass sich Bildaufnahmen mit und ohne diese Filter erzielen lassen, die anschließend miteinander verglichen werden können.

Das monochrome Filter, das Bandpassfilter oder das Polarisationsfilter werden elektronisch gesteuert.

Eine elektronische Steuerung dieser Filter wird durch Verwenden einer Spannung erzielt, durch die die optischen Eigenschaften dieser Filter gezielt eingestellt oder beeinflusst werden kann. Beispielsweise lässt sich hierdurch eine Wellenlänge, ein Wellenlängenbereich oder ein Polarisationswinkel der Filter gezielt steuern.

Diese elektronische Steuerung ermöglicht beispielsweise eine Selbstoptimierung des Kontrastes oder der Intensität zur Verbesserung des Informationsinhaltes des digitalen Bildes. Zu diesem Zweck kann eine elektronische Steuerungseinrichtung vorgesehen sein, die diese Steuerung durchführt.

In einer weiteren technisch vorteilhaften Ausführungsform des Bildaufnahmesystems umfasst das Bildaufnahmesystem eine Lichtquelle zum Anregen der Lumineszenz, die ausgebildet ist, kollimiertes Licht auszusenden. Dadurch wird beispielsweise der technische Vorteil erreicht, dass die Lichtstrahlen auf einen bestimmten Bereich gerichtet und konzentriert werden können.

In einer weiteren technisch vorteilhaften Ausführungsform des Bildaufnahmesystems liegt der Kollimationswinkel zwischen 0°-2° oder 0°-4°. Dadurch wird beispielsweise ebenfalls der technische Vorteil erreicht, dass die Lichtstrahlen auf einen bestimmten Bereich gerichtet und konzentriert werden können. In einer weiteren technisch vorteilhaften Ausführungsform des Bildaufnahmesystems umfasst das Bildaufnahmesystem eine Lichtquelle zum Anregen der Lumineszenz, die durch eine Leuchtdiode, eine Laserdiode oder einen Laser gebildet ist. Dadurch wird beispielsweise ebenfalls der technische Vorteil erreicht, dass besonders geeignete Lichtquellen verwendet werden.

Ein erfindungsgemässes Verfahren ist in Anspruch 12 definiert.

Dadurch ergeben sich die gleichen technischen Vorteile wie durch das Bildaufnahmesystem nach Anspruch 1.

In einer technisch vorteilhaften Ausführungsform des Verfahrens zur dentalen Bildaufnahme wird ein Kontrast oder eine Intensität des erfassten Lumineszenzbereiches im digitalen Bild erhöht. Dadurch wird beispielsweise ebenfalls der technische Vorteil erreicht, dass Lumineszenzbereiche noch deutlicher optisch wahrgenommen werden können.

In einer weiteren technisch vorteilhaften Ausführungsform des Verfahrens wird eine Tiefeninformation in das digitale Bild integriert, um die Basis des digitalen Bildes zu erhöhen. Dadurch wird beispielsweise ebenfalls der technische Vorteil erreicht, dass Lumineszenzbereiche noch deutlicher optisch wahrgenommen werden können.

In einer weiteren technisch vorteilhaften Ausführungsform des Verfahrens zur dentalen Bildaufnahme sendet die Lichtquelle monochromatisches, nicht-monochromatisches, nicht-polarisiertes oder polarisiertes Licht aus. Dadurch wird beispielsweise ebenfalls der technische Vorteil erreicht, dass geeignetes Licht zur Erfassung der Lumineszenz verwendet wird.

In einer weiteren technisch vorteilhaften Ausführungsform des Verfahrens zur dentalen Bildaufnahme sendet die Lichtquelle kollimiertes Licht aus. Dadurch wird beispielsweise ebenfalls der technische Vorteil erreicht, dass die Lichtstrahlen auf einen bestimmten Bereich gerichtet und konzentriert werden können.

In einer weiteren technisch vorteilhaften Ausführungsform des Verfahrens werden eine ausgesandte Wellenlänge, ein elektronisches Filter und/oder die elektronische Kamera derart gesteuert, dass eine automatische Erhöhung oder Optimierung des Kontrastes digitalen Bildes oder eine Erhöhung der Empfindlichkeit der Kamera erfolgt. Auf diesem Wege kann beispielsweise eine Selbstoptimierung des Kontrastes oder der Intensität des Lumineszenzbereiches erfolgen. Dadurch wird beispielsweise ebenfalls der technische Vorteil erreicht, dass Lumineszenzbereiche schnelle und deutlicher wahrgenommen werden können. Diese Selbstoptimierung kann von geeigneten Algorithmen vorgenommen werden, die die ausgesandte Wellenlänge, das elektronische Filter und/oder die elektronische Kamera auf Basis des erfassten Bildes steuern. Ausführungsbeispiele der Erfindung sind in den Zeichnungen dargestellt und werden im Folgenden näher beschrieben.

Es zeigen:
- Fig. 1: eine schematische Ansicht eines dentalen Bildaufnahmesystems; und
- Fig. 2: ein Blockdiagram eines Verfahrens zur dentalen Bildaufnahme.

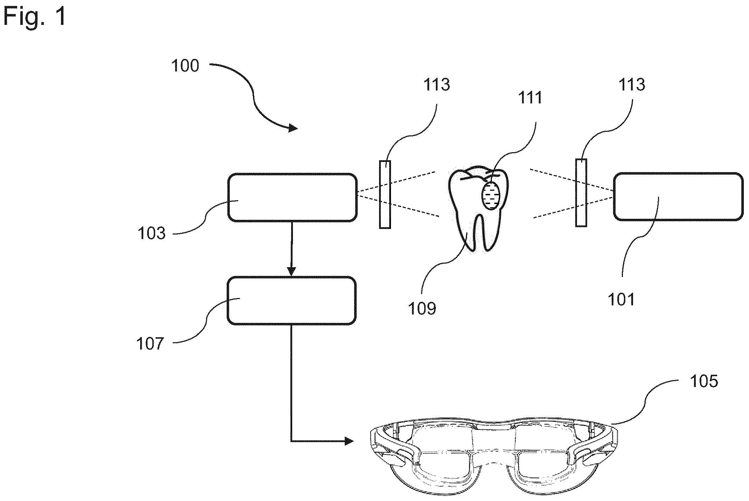

Fig. 1 zeigt eine schematische Ansicht eines dentalen Bildaufnahmesystems 100. Das Bildaufnahmesystem 100 dient zum erhalten von Bildaufnahmen aus der Mundhöhle eines Patienten. Das Bildaufnahmesystem 100 umfasst optional eine Lichtquelle 101 zum Anregen einer Lumineszenz im Inneren der Mundhöhle, eine elektronische Kamera 103 zum Erfassen eines digitalen Bildes eines Lumineszenzbereiches 111; und einer Datenbrille 105 zum Anzeigen des erfassten Bildes über einem visuell wahrgenommenen Bild. Durch eine digitale Verarbeitungseinrichtung kann der Lumineszenzbereich 111 in dem erfassten Bild optisch hervorgehoben oder verstärkt werden, so dass dieser besser erkannt wird.

Die Lumineszenz umfasst eine Fluoreszenz, eine Phosphoreszenz und ein Raman. Die Fluoreszenz ist die spontane Emission von Licht kurz nach der Anregung eines Materials durch elektronische Übergänge. Dabei ist das emittierte Licht in der Regel energieärmer als das vorher durch die Lichtquelle 101 absorbierte. Die Phosphoreszenz beschreibt ein längeres Nachleuchten nach der optischen Anregung. Die Raman ist die unelastische Streuung von Licht an Molekülen bezeichnet.

Durch die Fluoreszenz, Phosphoreszenz oder Raman lässt sich eine Porosität, eine Rauigkeit oder Glanzwerte von Zahnsubstanz sichtbar machen. Dadurch kann festgestellt werden, ob ein Zahn 109 kariös ist oder nicht oder in welchem anderen Zustand sich der Zahn befindet.

Die Lichtquelle 101 ist beispielsweise durch eine Leuchtdiode, eine Laserdiode oder einen Laser gebildet. Die Lichtquelle 101 kann Licht im Wellenlängenbereich von 300 nm bis 480 nm oder 330 nm bis 400 nm aussenden. Der Lichtstrahl der Lichtquelle kann kollimiert sein, d.h. im Wesentlichen parallel ausgerichtet sein, so dass sich das Licht auf einen bestimmten Bereich im Inneren der Mundhöhle konzentrieren lässt. Dies kann beispielsweise auf einfache Weise durch eine Kollimator- oder Sammellinse vor der Lichtquelle oder einen Laser als Lichtquelle 101 erreicht werden.

Die Lichtquelle 101 kann als separates Gerät ausgebildet sein, das nur zum Zweck der Beleuchtung vorgesehen ist oder kann in einem anderen zahnmedizinischen Behandlungswerkzeug intergiert sein, wie beispielsweise einem Scanner, einer Sonde oder einem Spiegel. Die Lichtquelle 101 kann monochromatisches, nicht-monochromatisches, nicht-polarisiertes oder polarisiertes Licht aussenden. Im Allgemeinen kann die Lichtquelle 101 jede Form von Licht aussenden, die eine Lumineszenz anregt.

Der Lumineszenzbereich 111 (ROI - Region of Interest) wird durch eine elektronische Kamera 103 mit einem CMOS-Chip aufgenommen, die ein digitales Bild der Lumineszenz erfasst und entsprechende Bilddaten erzeugt. Das digitale Bild und die Bilddaten können informationstechnisch aufbereitet werden, so dass der beabsichtigte Lumineszenzeffekt zusätzlich hervorgehoben wird. Die Aufbereitung des digitalen Bildes kann durch eine Bildanalyse durchgeführt werden, die eine durch maschinelles Lernen (Maschine Learning) erarbeitete Unterscheidung von visuell kaum unterscheidbaren Lumineszenzzonen innerhalb des Lumineszenzbereiches 111 zulässt.

Zudem kann ein monochromes Filter 113, ein Bandpassfilter 113 oder ein Polarisationsfilter 113 in den Strahlengang der Kamera 103 bewegbar oder einklappbar sein, beispielsweise vor der Kamera 103 und/oder vor der Lichtquelle 101. Ein monochromes Filter lässt lediglich Licht einer bestimmten Wellenlänge passieren. Ein Bandpassfilter ist ein Filter, das nur Licht eines bestimmten Frequenzbereiches passieren lässt. Ein Polarisationsfilter lässt nur Licht einer bestimmten Polarisationsrichtung passieren.

Auf diese Weise lassen sich Bildaufnahmen mit und ohne diese Filter 113 erzielen, die anschließend miteinander verglichen werden können. Die erhaltenen Bilder können Gegenstand einer Differenzanalyse sein.

Die elektronische Kamera 103 kann beispielsweise Wellenlängen im kurzwelligen Lichtbereich unter 400 nm erfassen oder im längerwelligen Bereich über 700 nm, die vom Auge nicht mehr wahrgenommen werden können. Dadurch lassen sich auch nicht sichtbare Fluoreszenz-, Phosphoreszenz- oder Ramaneffekte erfassen.

Eine Berechnungseinrichtung 107 des dentalen Bildaufnahmesystems 100 kann die Aufbereitung der Bilddaten durchführen und den Kontrast oder die Intensität des erfassten Lumineszenzbereiches im digitalen Bild zu erhöhen. Hierzu können Lumineszenzbereiche 111 im digitalen Bild identifiziert werden und mittels Grafikalgorithmen bearbeitet werden. So ist es möglich, schwache Fluoreszenzeffekte, Phosphoreszenzeffekte oder Ramaneffekte zu verstärken oder mit anderen Farbewerten zu versehen, die besser wahrnehmbar sind. Eine Identifikation des Lumineszenzbereiches 111 kann über die Erfassung von charakteristischen Wellenlängen/Farben der Lumineszenz im aufgenommenen Bild erfolgen.

Die Berechnungseinrichtung 107 ist beispielsweise durch ein Softwaremodul gebildet, das durch einen Prozessor ausgeführt wird. Die ursprünglichen und aufbereiteten Bilddaten lassen sich in einem digitalen Speicher ablegen.

Zudem kann die Berechnungseinrichtung 107 eine Tiefeninformation in das digitale Bild integrieren, um die Basis des digitalen Bildes zu erhöhen. Die Tiefeninformation kann durch Grauwerte widergegeben werden und eine räumliche oder dreidimensionale Situation des Zahnes wiedergeben. Die Tiefeninformation kann beispielsweise ein Bild sein, das durch optische Kohärenztomografie (OCT - Optical Coherence Tomography) oder ein Röntgenverfahren gewonnen wird. Diese Tiefeninformation kann zusätzlich auf die Datenbrille 105 projiziert werden. Die erfassten Lumineszenzbereiche können auf der Tiefeninformation superponiert werden. Dadurch werden zusätzliche Information geliefert, die zu einer besseren Diagnose führen.

Anschließend kann das Bild oder die Bilddaten dem Benutzer auf einer Datenbrille 105 dargestellt werden, so dass Lumineszenzbereiche 111 besser wahrgenommen werden können. Die Datenbrille 105 erhält die Bilddaten des aufgenommenen Bildes und ist ein als Brille getragenes elektronisches Gerät, mit dem einem Benutzer zusätzlich zur natürlichen visuellen Wahrnehmung des Zahnbereichs Fluoreszenzeffekte, Phosphoreszenzeffekte oder Ramaneffekte optisch angezeigt werden können.

Die Datenbrille 105 kann durch die Anzeigeeinrichtung zusätzliche Informationen auf einem Bild überlagern, das von dem Auge des Trägers wahrgenommen wird. Zur bildlichen Überlagerung auf dem visuell wahrgenommenen Bild können spezielle Softwarealgorithmen verwendet werden. Die Anzeigeeinrichtung der Datenbrille 105 umfasst beispielsweise einen augennahen Bildschirm oder einen Projektor zur direkten Projektion auf der Netzhaut. In entsprechende Weise kann dies auch auf einem tragbaren Gerät erfolgen.

Zudem kann eine Zoom-Funktion in das Bildaufnahmesystem 100 integriert sein, um das erfasste Bild über dem visuell wahrgenommenen Bild vergrößern zu können und so Lumineszenzbereiche 111 besser sichtbar zu machen. Eine Steuerung des Bildaufnahmesystems 100 kann durch die Datenbrille 105 mittels Stimme oder Bewegung erfolgen, so dass eine Selbstoptimierung der Intensität oder des Kontrastes des erfassten Bildes vorgenommen werden kann.

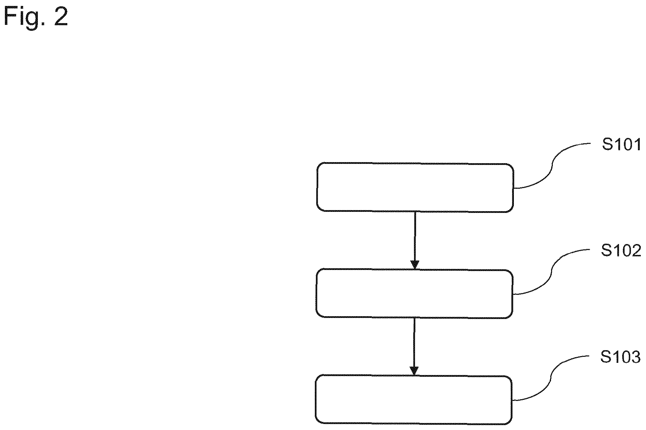

Fig. 2 zeigt ein Blockdiagram eines Verfahrens zur dentalen Bildaufnahme. Das Verfahren umfasst den Schritt S101 eines Anregens der Lumineszenz im Inneren einer Mundhöhle mittels der Lichtquelle 101, den Schritt S102 eines Erfassens des digitalen Bildes der Lumineszenz mittels einer elektronischen Kamera 103; und den Schritt S103 eines Anzeigens des erfassten Bildes über einem visuell wahrgenommenen Bild auf einer Datenbrille 105. Dadurch können Lumineszenzeffekte beim Betrachten des Lumineszenzbereiches 111 deutlich sichtbar gemacht werden.

Alle Verfahrensschritte können durch Vorrichtungen implementiert werden, die zum Ausführen des jeweiligen Verfahrensschrittes geeignet sind.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Dentales Bildaufnahmesystem
- 101: Lichtquelle
- 103: elektronische Kamera
- 105: Datenbrille
- 107: Berechnungseinrichtung
- 109: Zahn
- 111: Lumineszenzbereich
- 113: monochromes Filter/Bandpassfilter/Polarisationsfilter

## Patentansprüche

1. Dentales Bildaufnahmesystem (100), mit:
einer elektronischen Kamera (103) zum Erfassen eines digitalen Bildes eines Lumineszenzbereiches (111), die ein monochromes Filter (113), ein Bandpassfilter (113) oder ein Polarisationsfilter (113) umfasst; und
einer Datenbrille (105) zum Anzeigen des erfassten Bildes über einem visuell wahrgenommenen Bild;
eine elektronische Steuerung, die durch Verwenden einer Spannung die optischen Eigenschaft des jeweiligen Filters gezielt einstellen oder beeinflussen kann.

2. Bildaufnahmesystem (100) nach Anspruch 1, wobei das dentale Bildaufnahmesystem (100) eine Berechnungseinrichtung (107) umfasst, die ausgebildet ist, einen Kontrast oder eine Intensität des erfassten Lumineszenzbereiches (111) im digitalen Bild zu erhöhen.

3. Bildaufnahmesystem (100) nach einem der vorangehenden Ansprüche, wobei das dentale Bildaufnahmesystem (100) eine Berechnungseinrichtung (107) umfasst, die ausgebildet ist, eine Tiefeninformation in das digitale Bild zu integrieren, um die Basis des digitalen Bildes zu erhöhen.

4. Bildaufnahmesystem (100) nach einem der vorangehenden Ansprüche, wobei eine Lichtquelle (101) zum Anregen einer Lumineszenz und/oder die elektronische Kamera (103) in der Datenbrille (105) oder in einer dentalen Behandlungsvorrichtung (109) integriert ist.

5. Bildaufnahmesystem (100) nach einem der vorangehenden Ansprüche, wobei die Lichtquelle (101) ausgebildet ist, monochromatisches, nicht-monochromatisches, nicht-polarisiertes oder polarisiertes Licht auszusenden.

6. Bildaufnahmesystem (100) nach Anspruch 5, wobei das Licht eine Wellenlänge im Bereich von 300 bis 480 nm aufweist.

7. Bildaufnahmesystem (100) nach einem der vorangehenden Ansprüche, wobei die Lichtquelle (101) ein monochromes Filter (113), ein Bandpassfilter (113) oder ein Polarisationsfilter (113) umfasst.

8. Bildaufnahmesystem (100) nach Anspruch 7, wobei das monochrome Filter (113), das Bandpassfilter (113) oder das Polarisationsfilter (113) bedarfsweise in den Strahlengang der Lichtquelle (101) bewegbar oder steuerbar ist.

9. Bildaufnahmesystem (100) nach einem der vorangehenden Ansprüche, wobei das Bildaufnahmesystem (100) eine Lichtquelle (101) zum Anregen der Lumineszenz umfasst, die ausgebildet ist, kollimiertes Licht auszusenden.

10. Bildaufnahmesystem (100) nach Anspruch 9, wobei der Kollimationswinkel zwischen 0°-2° oder 0°-4° liegt.

11. Bildaufnahmesystem (100) nach einem der vorangehenden Ansprüche, wobei das Bildaufnahmesystem (100) eine Lichtquelle (101) zum Anregen der Lumineszenz umfasst, die durch eine Leuchtdiode, eine Laserdiode oder einen Laser gebildet ist.

12. Verfahren zur dentalen Bildaufnahme (100), mit den Schritten:
Erfassen (S102) eines digitalen Bildes eines Lumineszenzbereiches (111) mittels einer elektronischen Kamera (103), die ein monochromes Filter (113), ein Bandpassfilter (113) oder ein Polarisationsfilter (113) umfasst;
elektronisches Steuern des monochromen Filters (113), des Bandpassfilters (113) oder des Polarisationsfilters (113) durch Verwenden einer Spannung, durch die die optischen Eigenschaften dieser Filter gezielt eingestellt oder beeinflusst werden; und
Anzeigen (S103) des erfassten Bildes über einem visuell wahrgenommenen Bild auf einer Datenbrille (105).

13. Verfahren nach Anspruch 12, wobei ein Kontrast oder eine Intensität des erfassten Lumineszenzbereiches (111) im digitalen Bild erhöht wird.

14. Verfahren nach einem der Ansprüche 12 oder 13, wobei eine Tiefeninformation in das digitale Bild integriert wird, um die Basis des digitalen Bildes zu erhöhen.

15. Verfahren nach einem der Ansprüche 12 bis 14, wobei eine ausgesandte Wellenlänge, ein elektronisches Filter (113) und/oder die elektronische Kamera (103) derart gesteuert werden, dass eine automatische Erhöhung des Kontrastes des digitalen Bildes oder eine Erhöhung der Empfindlichkeit der Kamera (103) erfolgt.

## Claims

1. A dental imaging system (100), comprising:
an electronic camera (103) for capturing a digital image of a luminescence region (111) comprising a monochrome filter (113), a bandpass filter (113) or a polarisation filter (113); and
data glasses (105) for displaying the captured image on top of a visually perceived image;
an electronic control which can purposefully adjust or influence the optical properties of the respective filter by using a voltage.

2. The imaging system (100) as claimed in claim 1, wherein the dental imaging system (100) comprises a calculation device (107) which is configured to increase a contrast or an intensity of the captured luminescence region (111) in the digital image.

3. The imaging system (100) as claimed in any one of the preceding claims, wherein the dental imaging system (100) comprises a calculation device (107) which is configured to integrate depth information into the digital image in order to increase the basis of the digital image.

4. The imaging system (100) as claimed in any one of the preceding claims, wherein a light source (101) for exciting luminescence and/or the electronic camera (103) is integrated in the data glasses (105) or in a dental treatment device (109).

5. The imaging system (100) as claimed in any one of the preceding claims, wherein the light source (101) is configured to emit monochromatic, non-monochromatic, non-polarised or polarised light.

6. The imaging system (100) as claimed in claim 5, wherein the light has a wavelength in the range of 300 to 480 nm.

7. The imaging system (100) as claimed in any one of the preceding claims, wherein the light source (101) comprises a monochrome filter (113), a bandpass filter (113) or a polarisation filter (113).

8. The imaging system (100) as claimed in claim 7, wherein the monochrome filter (113), the bandpass filter (113) or the polarisation filter (113) can be controlled or can be moved as required into the beam path of the light source (101) .

9. The imaging system (100) as claimed in any one of the preceding claims, wherein the imaging system (100) comprises a light source (101) for exciting luminescence, which is configured to emit collimated light.

10. The imaging system (100) as claimed in claim 9, wherein the collimation angle is between 0°-2° or 0°-4°.

11. The imaging system (100) as claimed in any one of the preceding claims, wherein the imaging system (100) comprises a light source (101) for exciting luminescence, which is formed by a light-emitting diode, a laser diode or a laser.

12. A method for dental imaging (100), comprising the steps of:
capturing (S102) a digital image of a luminescence region (111) by means of an electronic camera (103) comprising a monochrome filter (113), a bandpass filter (113) or a polarisation filter (113);
electronically controlling the monochrome filter (113), the bandpass filter (113) or the polarisation filter (113) by using a voltage by means of which the optical properties of these filters are purposefully adjusted or influenced; and
displaying (S103) the captured image on top of a visually perceived image on data glasses (105).

13. The method as claimed in claim 12, wherein a contrast or an intensity of the captured luminescence region (111) in the digital image is increased.

14. The method as claimed in any one of claims 12 or 13,
wherein depth information is integrated into the digital image in order to increase the basis of the digital image.

15. The method as claimed in any one of claims 12 to 14,
wherein an emitted wavelength, an electronic filter (113) and/or the electronic camera (103) are controlled in such a way that an automatic increase in the contrast of the digital image or an increase in the sensitivity of the camera (103) takes place.

## Revendications

1. Système de capture d'images dentaires (100), comprenant :
une caméra électronique (103) pour l'acquisition d'une image numérique d'une zone de luminescence (111), comprenant un filtre monochrome (113), un filtre passe-bande (113) ou un filtre polarisant (113) ; et
des lunettes intelligentes (105) pour afficher l'image acquise au-dessus d'une image perçue visuellement ;
une commande électronique qui, en utilisant une tension, peut régler ou influencer de manière sélective la propriété optique du filtre respectif.

2. Système de capture d'image (100) selon la revendication 1, où le système de capture d'image dentaire (100) comprend un dispositif de calcul (107) qui est conçu pour augmenter un contraste ou une intensité de la zone de luminescence acquise (111) dans l'image numérique.

3. Système de capture d'image (100) selon l'une des
revendications précédentes, où le système de capture d'image dentaire (100) comprend un dispositif de calcul (107) configuré pour intégrer une information de profondeur dans l'image numérique afin d'augmenter la base de l'image numérique.

4. Système de capture d'image (100) selon l'une des
revendications précédentes, où une source de lumière (101) pour exciter une luminescence et/ou la caméra électronique (103) est intégrée dans les lunettes intelligentes (105) ou dans un dispositif de traitement dentaire (109).

5. Système de capture d'image (100) selon l'une des
revendications précédentes, où la source lumineuse (101) est configurée pour émettre une lumière monochromatique, non monochromatique, non polarisée ou polarisée.

6. Système de capture d'image (100) selon la revendication 5, où la lumière présente une longueur d'onde comprise entre 300 et 480 nm.

7. Système de capture d'image (100) selon l'une des
revendications précédentes, où la source lumineuse (101) comprend un filtre monochrome (113), un filtre passe-bande (113) ou un filtre polarisant (113).

8. Système de capture d'image (100) selon la revendication 7, où le filtre monochrome (113), le filtre passe-bande (113) ou le filtre de polarisation (113) peut être déplacé ou commandé à la demande dans le trajet optique de la source lumineuse (101) .

9. Système de capture d'image (100) selon l'une des
revendications précédentes, où le système de capture d'image (100) comprend une source de lumière (101) pour exciter la luminescence, configurée pour émettre une lumière collimatée.

10. Système de capture d'image (100) selon la revendication 9, où l'angle de collimation est compris entre 0°-2° ou 0°-4°.

11. Système de capture d'image (100) selon l'une des
revendications précédentes, où le système de capture d'image (100) comprend une source de lumière (101) pour exciter la luminescence, qui est formée par une diode électroluminescente, une diode laser ou un laser.

12. Procédé d'imagerie dentaire (100), comprenant les étapes suivantes :
acquisition (S102) d'une image numérique d'une zone de luminescence (111) au moyen d'une caméra électronique (103) comprenant un filtre monochrome (113), un filtre passe-bande (113) ou un filtre polarisant (113) ;
commande électronique du filtre monochrome (113), du filtre passe-bande (113) ou du filtre de polarisation (113) en utilisant une tension par laquelle les propriétés optiques de ces filtres sont sélectivement ajustées ou influencées ; et
affichage (S103) de l'image acquise au-dessus d'une image perçue visuellement sur des lunettes intelligentes (105).

13. Procédé selon la revendication 12, où un contraste ou une intensité de la zone de luminescence détectée (111) est augmenté dans l'image numérique.

14. Procédé selon l'une des revendications 12 ou 13, où une donnée de profondeur est intégrée dans l'image numérique pour augmenter la base de l'image numérique.

15. Procédé selon l'une des revendications 12 à 14, où une longueur d'onde émise, un filtre électronique (113) et/ou la caméra électronique (103) sont commandés de manière à réaliser une augmentation automatique du contraste de l'image numérique ou une augmentation de la sensibilité de la caméra (103) .
